(19) Europäisches Patentamt
European Patent Office
Office européen des brevets

(11) EP 0 906 943 A1

(12) **EUROPEAN PATENT APPLICATION**
published in accordance with Art. 158(3) EPC

(43) Date of publication:
**07.04.1999 Bulletin 1999/14**

(21) Application number: **97924283.1**

(22) Date of filing: **30.05.1997**

(51) Int. Cl.[6]: **C09D 11/00**, C09B 47/20, C07F 1/08, C07D 487/22

(86) International application number:
**PCT/JP97/01859**

(87) International publication number:
**WO 97/45494 (04.12.1997 Gazette 1997/52)**

(84) Designated Contracting States:
**CH DE FR GB LI**

(30) Priority: **30.05.1996 JP 157383/96**

(71) Applicant:
**NIPPON KAYAKU CO., LTD.**
**Chiyoda-ku, Tokyo 102 (JP)**

(72) Inventors:
- **OJIMA, Masayoshi**
  **Yono-shi, Saitama-ken 338 (JP)**
- **KAJIURA, Noriko**
  **Katsushika-ku, Tokyo 125 (JP)**

(74) Representative:
**Türk, Gille, Hrabal**
**Brucknerstrasse 20**
**40593 Düsseldorf (DE)**

(54) **RECORDING FLUIDS CONTAINING COPPER-PHTHALOCYANINE COMPOUNDS**

(57) The present invention relates to a recording solution which is characterized in containing a copper phthalocyanine compound represented by the following formula (I)

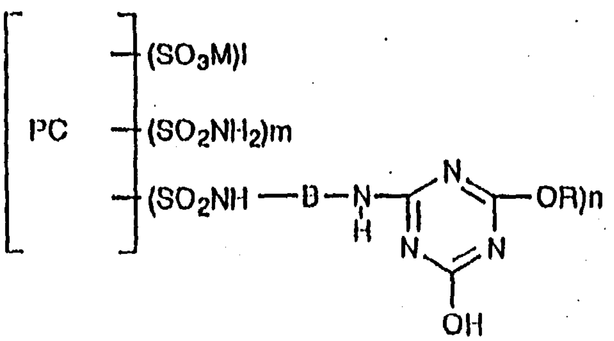

( I )

(In the formula(I), PC is a copper phthalocyanine skeleton; B is a $C_2$~$C_4$ alkylene group or a phenylene group which may be substituted with carboxyl group(s) or with sulfonic acid group(s); R is a lower alkyl group or a lower alkoxy lower alkyl group; M is hydrogen atom, an alkali metal or an alkali earth metal; l is 1~3; m is 0~2; and n is 1 or 2 with a proviso that (l + m + n) is 2~4).

The recording solution of the present invention is a recording solution which gives image of high concentration and clear color tone having a good storage stability, particularly in terms of water resistance.

EP 0 906 943 A1

## Description

### Technical Field

The present invention relates to a novel use of copper phthalocyanine compounds. More particularly, it relates to a recording solution containing copper phthalocyanine compounds which is suitable for printing, writing implements, recording instruments and stamp, particularly for ink jet recording.

### Background Art

With regard to recording methods for color image, there are melt transfer recording, sublimatic transfer recording, electrophotographic recording, ink jet recording, etc. and, among them, an ink jet recording method has made a quick spread since it has many advantages in terms of quality of image, cost, convenience, etc.

With respect to the recording solution for the ink jet recording method, that which contains water-soluble dyes such as direct dyes and acid dyes have been used in many cases. Characteristics requested to said recording solution are as follows.

(1) Quality of the print is to be high. Thus, contrast of the printed image is to be high and the image is to be clear without running.
(2) Stability of the printed image is to be high. Thus, it is to have good resistance to light, particularly to water and also to rubbing.
(3) The ink is to have good injection characteristics. Thus, it is not to separate the crystals for clogging the nozzle during a storage for long term or by chemical changes, etc. upon use. Further, viscosity, surface tension, specific conductivity and density of the ink are to be within appropriate ranges.

Many of the above-mentioned characteristics are much dependent upon the characteristics of the dye contained in the recording solution. Among the above-mentioned characteristics, water resistance will be becoming more and more important in future. This is because recording paper is used after subjecting to a process such as prevention of "running(bleeding)" due to the fact that no highly water-resistant ink is available at present. Now it is believed that, in view of the problem of environmental protection, recovery of waste paper is becoming active and that there will be a demand for the recording solution having good water resistance on such a recycled paper.

An object of the present invention is to offer a recording solution which gives high image concentrations and clear color tone to various kinds of ordinary paper and, in addition, gives the image having good storage stability, particularly water resistance.

### Disclosure of the invention

An intensive study has been conducted in order to find a recording solution having a high image concentration, giving clear color tone and exhibiting good water resistance and, as a result, the present invention has been achieved. Thus, the present invention relates to:

(1) a recording solution which is characterized in containing a copper phthalocyanine compound represented by the following formula (I)

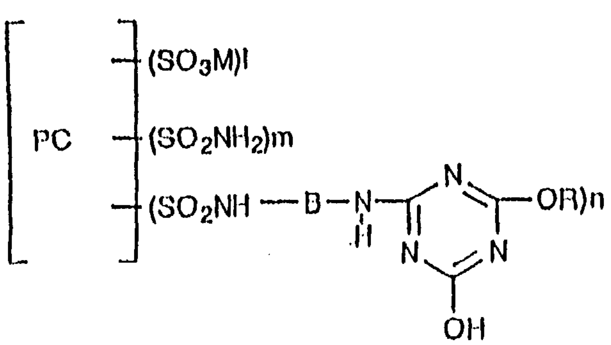

( I )

(In the formula, PC is a copper phthalocyanine skeleton; B is $C_2$~$C_4$ alkylene group or phenylene group which may

be substituted with carboxyl group or with sulfonic acid group; R is lower alkyl group or lower alkoxy lower alkyl group; M is hydrogen atom, alkali metal or alkali earth metal; l is 1~3; m is 0~2; and n is 1 or 2 with a proviso that (l + m + n) is 2~4);

(2) a recording solution according to (1) in which the content of the copper phthalocyanine in the recording solution is 0.1~15% by weight;

(3) a recording solution according to (1) or (2) mentioned above in which viscosity of the recording solution at 25°C is 1~20 mPa.s and surface tension thereof is not less than 30 mN/m; and

(4) a recording solution according to any of (1) to(3) mentioned above in which said recording solution is for ink jet recording.

Best Modes for Conducting the Invention

The present invention will now be further illustrated as hereunder.

The compound of the formula (I) may be manufactured by a method mentioned in the Japanese Laid-Open Patent Publication Sho-61/275361 by, for example, the following manner.

Thus, methanol is made to react with cyanuric chloride in methanol in the presence of an alkaline agent such as sodium hydrogen carbonate at around 0°C to give 2-methoxy-4,6-dichloro-1,3,5-triazine. This is made to react with ethylenediamine dihydrochloride in the presence of sodium carbonate under such a reaction condition that pH is about 5 and temperature is 40°C to give 2-methoxy-4-(2-aminoethylamino)-6-chloro-1,3,5-triazine. In the meanwhile, copper phthalocyanine is made to react with chlorosulfonic acid to give copper phthalocyanine chlorosulfonate. Then the above-prepared 2-methoxy-4-(2-aminoethylamino)-6-chloro-1,3,5-triazine is made to react with this copper phthalocyanine chlorosulfonate in the presence of sodium hydroxide under such a reaction condition that pH is 10.5 and temperature is 97°C to give the compound of the formula (I) which is used in the present invention.

M in the compound of the formula (I) may be an alkali metal or an alkali earth metal such as lithium, sodium, potassium or calcium and, preferably, it is sodium or lithium. An example of the group R in the same formula is a $C_1$~$C_4$ linear or branched alkyl group and its specific example are methyl group, ethyl group, butyl group and isopropyl group etc.. Methyl group and ethyl group are preferred. Furthermore, an example of the lower alkoxy lower alkyl group represented by R is a $C_1$~$C_4$ linear or a branched alkyl group which is substituted with $C_1$~$C_4$ linear or branched alkoxy group(s) and its specific examples are methoxymethyl group, methoxyethyl group, ethoxyethyl group, methoxyisopropyl group and ethoxybutyl group. Methoxymethyl group and methoxyethyl group are preferred. The $C_2$~$C_4$ alkylene group in group B may be either linear or branched chain and is, for example, ethylene group, propylene group or butylene group. With regard to the phenylene group, any of o-phenylene group, m-phenylene group and p-phenylene group may be used although m-phenylene group or p-phenylene group is preferred. Such a phenylene group may be substituted with carboxyl group(s) or sulfonic acid group(s) and there is no particular limitation for the position(s) and the number(s) of said substituent. Preferred numbers for l, m and n are that 1 or 2 for l; 0 for m; and 2 for n.

Specific examples of the copper phthalocyanine compound represented by the formula (I) used in the present invention are as shown in the following Table 1 although said compound is not limited to those exemplified ones only.

Table 1

| Compound No. | -B- | -R | M | l | m | n |
|---|---|---|---|---|---|---|
| 1 | $-C_2H_4-$ | $-CH_3$ | H | 1 | 0 | 2 |
| 2 | $-C_2H_4-$ | $-CH_3$ | H | 1 | 1 | 2 |
| 3 | $-C_2H_4-$ | $-C_2H_5$ | Na | 1 | 0 | 2 |
| 4 | $-C_4H_8(n)-$ | $-CH_3$ | Li | 2 | 0 | 2 |
| 5 | $-C_2H_4-$ | $-C_2H_4OCH_3$ | H | 1 | 0 | 2 |
| 6 | p-$C_6H_3$-2-($SO_3H$)- | $-C_2H_5$ | Ca | 1 | 0 | 2 |
| 7 | m-$C_6H_3$-3-(COOH)- | $-C_2H_5$ | H | 1 | 0 | 2 |

In the recording solution of the present invention, content of the copper phthalocyanine compound of the formula (I) in the recording solution is usually 0.1~15% by weight or, preferably, 0.5~10% by weight. Incidentally, the copper phthalocyanine compound of the formula (I) may be used either solely or jointly by mixing two or more. In addition, compound (dye) which is other than that of the formula (I) may be contained therein within such an extent that it does not deteriorate the gist of the present invention.

In the recording solution of the present invention, water or a mixed solvent consisting of water and a water-soluble organic solvent may be used as a medium.

Specific examples of the usable water-soluble organic solvent are $C_1$~$C_5$ alcohols such as methyl alcohol, ethyl alcohol, n-propyl alcohol, isopropyl alcohol, n-butyl alcohol, sec-butyl alcohol, tert-butyl alcohol and isobutyl alcohol; amides such as dimethylformamide and dimethylacetamide; ketones such as acetone; ethers such as dioxane; $C_2$~$C_6$ alkylene glycols such as ethylene glycol, propylene glycol, butylene glycol, triethylene glycol and hexylene glycol; polyalkyl glycols such as polyethylene glycol and polypropylene glycol; glycerol; polyhydric alcohol alkyl ethers such as ethylene glycol monomethyl ether, ethylene glycol monoethyl ether, diethylene glycol monomethyl ether and diethylene glycol monoethyl ether; polyhydric alcohol dialkyl ethers such as triethylene glycol dimethyl ether and triethylene glycol diethyl ether; sulfolane; N-methyl-2-pyrrolidone; and 1,3-dimethyl-2-imidazolidinone. Two or more of those water-soluble organic solvents may be used by mixing them. The ratio of water to the water-soluble organic solvent when water and the water-soluble organic solvent are used by mixing them is usually from 10:1 to 1.5:1. Content of the medium consisting of water or of water and the water-soluble organic solvent in the recording solution is preferably 2~85% by weight and, more preferably, 3~70% by weight.

Preferred viscosity of the recording solution of the present invention at 25°C is 1~20 mPa.s or, more preferably, 1~15 mPa.s while preferred surface tension of said solution at 25°C is not less than 30 mN/m or, more preferably, not less than 40 mN/m. In addition, it is preferred that the recording solution of the present invention is adjusted to pH 6~11 or, preferably, pH 7~9 using ammonia or the like.

In order to further improve the quick drying after printing and the quality of the printed image, the recording solution of the present invention may contain 0.1~10% by weight of a compound such as urea, thiourea, semicarbazide, etc. furthermore 0.001~5.0% by weight of a surface-active agent.

The recording solution of the present invention can be manufactured by mixing and stirring the above-mentioned components. If necessary, after mixing and stirring as above, the recording solution of the present invention may be filtered to remove contaminants. Pore size of the filter in that case is preferably about 0.3~0.5μm.

The recording solution of the present invention may be used for printing, writing implements, recording instruments, stamp, etc. and preferred use is for ink jet recording. In conducting the printing (recording) by the use of the recording solution of the present invention by an ink jet means, a method which is known *per se* may be used.

The recording solution of the present invention gives prints of high image concentration and clear color tone to various kinds of ordinary paper and, in addition, the resulting print (image) exhibits a good storage stability, particularly water resistance. In addition, the recording solution of the present invention gives good print to recycled paper as well.

The present invention will now be further illustrated by way of the examples although the present invention is not limited thereto. In the examples, the terms "part(s)" and "%" are "part(s) by weight" and "% by weight", respectively.

Example 1.

Water was added to 10 parts of diethylene glycol, 4 parts of isopropylene glycol and 3 parts of the above-mentioned compound No.1, the pH was adjusted to 8.5 by aqueous ammonia solution and water was added to make the total weight 100 parts. The resulting mixed product was stirred to dissolve homogenoously. After that, it was filtered through a filter having a pore size of 0.5μm to give a recording solution of the present invention.

Recording was conducted by the above-prepared recording solution on an electrophotographic paper (neutral paper; manufactured by Xerox) using an ink jet printer (Disk Jet HP5166A; manufactured by Hewlett-Packard) to give a print in cyan color.

This was tested in terms of light resistance, water resistance and storage stability of the recording solution. Test method and criterion for each of them are as follows.

(1) Light Resistance Test

Irradiation was conducted for 100 hours using a xenon Fade-Ometer (manufactured by Suga Shikenki) and residual rate of OD as defined below was measured and judged.

$$\text{Residual Rate of OD} = [(\text{OD after Irradiation})/(\text{OD before Irradiation})] \times 100\ (\%)$$

The OD of the printed part (maximum absorption wave length: 673nm) was measured by a Macbeth densitometer (Color Eye 3000; manufactured by Macbeth). As a result, the residual rate of OD was 98%.

(2) Water Resistance

The above-prepared printed image was dipped in tap water for 5 minutes. After that, it was taken out and the degree of running of the printed part was judged by naked eye. In the meanwhile, the residual rate of OD as defined below was measured.

$$\text{Residual Rate of OD} = [(\text{OD after Dipping})/(\text{OD before Dipping})] \times 100\ (\%)$$

The result was that no running was noted at all and the residual rate of OD was 99%.

(3) Storage Stability of the Recording Solution

The recording solution was placed in a tightly closed container and stored at 5°C and 50°C for one month. After that, precipitation of insoluble matter was checked by naked eye and, in addition, nozzle of the ink jet printer was tested whether or not it was clogged. As a result, precipitation of the insoluble matter was not noted and, as recorded by the ink jet printer, no clogging of the nozzle was noted as well.

Example 2.

Water was added to 10 parts of diethylene glycol, 5 parts of N-methyl-2-pyrrolidone and 3 parts of the above-mentioned compound No.2, the pH was adjusted to 9 by aqueous ammonia solution and water was added to make the total weight 100 parts. The resulting mixed product was stirred to dissolve homogeneously. After that, it was filtered through a filter having a pore size of 0.3μm to give a recording solution of the present invention. The resulting recording solution was used for conducting the recording and printing by the same manner as in Example 1 and then each of the above-mentioned tests was conducted.

As a result, the residual rate of OD in the light resistance test (maximum absorption wave length: 672 nm) was 98.5% and the residual rate of OD in the water resistance test was 99%. In addition, neither precipitation of insoluble matter nor clogging of the nozzle was noted in the storage stability test of the recording solution.

Examples 3~7.

Compound No. 3, 4, 5, 6 or 7 was used instead of the compound No. 1 used in Example 1 or the compound No. 2 used in Example 2 and the same operation as in Example 1 or 2 was conducted to give the recording solution.

Industrial Applicability

The recording solution obtained by the present invention gave high image concentrations and clear cyan color image even in the case of ordinary paper and, in addition, it gave image of good storage stability, particularly in terms of water resistance. Accordingly, the recording solution of the present invention is well capable of being used as a recording solution for ink jet printing.

## Claims

1. A recording solution which is characterized in containing a copper phthalocyanine compound represented by the following formula (I)

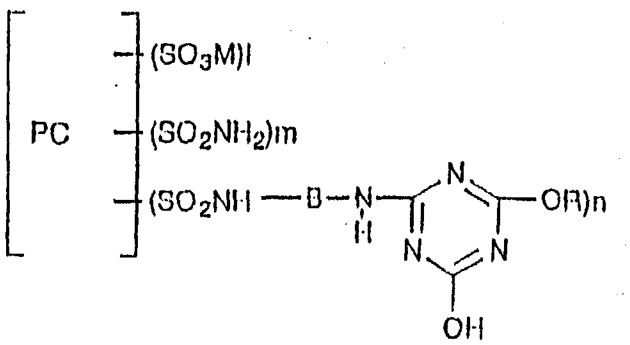

(I)

(In the formula (I), PC is a copper phthalocyanine skeleton; B is a $C_2$~$C_4$ alkylene group or a phenylene group which bay be substituted with carboxyl group(s) or with sulfonic acid group(s); R is a lower alkyl group or lower alkoxy lower alkyl group; M is hydrogen atom, an alkali metal or an alkali earth metal; l is 1~3; m is 0~2; and n is 1 or 2 with a proviso that (l + m + n) is 2~4).

2. A recording solution according to claim 1 in which the content of the copper phthalocyanine in the recording solution is 0.1~15% by weight.

3. A recording solution according to claim 1 or 2 in which viscosity of the recording solution at 25°C is 1~20 mPa.s and surface tension thereof is not less than 30 mN/m.

4. A recording solution according to any of claims 1 to 3 in which said recording solution is for ink jet recording.

## INTERNATIONAL SEARCH REPORT

International application No. PCT/JP97/01859

A. CLASSIFICATION OF SUBJECT MATTER

Int. $Cl^6$ C09D11/00, C09B47/20, C07F1/08, C07D487/22

According to International Patent Classification (IPC) or to both national classification and IPC

B. FIELDS SEARCHED

Minimum documentation searched (classification system followed by classification symbols)

Int. $Cl^6$ C09D11/00, C09B47/20, C07F1/08, C07D487/22

Documentation searched other than minimum documentation to the extent that such documents are included in the fields searched

Jitsuyo Shinan Koho 1926 - 1996
Kokai Jitsuyo Shinan Koho 1971 - 1997
Toroku Jitsuyo Shinan Koho 1994 - 1997
Jitsuyo Shinan Keisai Koho 1996 - 1997

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| X | JP, 3-185080, A (Canon Inc.), August 13, 1991 (13. 08. 91), Claim; page 6, lower left column, specific example Nos. 23, 24; page 8, upper left column, lines 12 to 16; page 9, upper left column, lines 3 to 7 & EP, 418792, A1 & US, 5123960, A & DE, 69021306, E | 1 - 4 |
| A | JP, 3-103484, A (Canon Inc.), April 30, 1991 (30. 04. 91), Claim (Family: none) | 1 - 4 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| August 14, 1997 (14. 08. 97) | August 26, 1997 (26. 08. 97) |
| Name and mailing address of the ISA/ Japanese Patent Office | Authorized officer |
| Facsimile No. | Telephone No. |

Form PCT/ISA/210 (second sheet) (July 1992)